# EUROPEAN PATENT SPECIFICATION

(11) **EP 3 517 450 B1**
(45) Date of publication and mention of the grant of the patent: **11.03.2026**
(21) Application number: 17853090.3
(22) Date of filing: 20.09.2017
(51) Int. Cl.: B67C 7/00, B65B 1/02, G01N 21/90, G03B 17/08, G03B 15/00, G03B 15/03, B29C 49/78

(54) **ASEPTIC FILLING MACHINE**
MASCHINE ZUM ASEPTISCHEN BEFÜLLEN
MACHINE DE REMPLISSAGE ASEPTIQUE

(30) Priority: 23.09.2016 JP 2016185809
(43) Date of publication of application: 31.07.2019
(73) Proprietor: Dai Nippon Printing Co., Ltd., Tokyo 162-8001 (JP)
(72) Inventor: TAKAKU, Hitoshi, Tokyo 162-8001 (JP)
(74) Representative: Müller-Boré & Partner Patentanwälte PartG mbB
(86) International application number: PCT/JP2017/033946
(87) International publication number: WO 2018/056320

(56) References cited:
- EP-A1- 2 653 395
- EP-A1- 3 070 010
- DE-A1- 102004 054 859
- DE-A1- 102014 216 576
- JP-A- 2002 221 748
- JP-A- 2010 155 631
- JP-A- 2013 035 561
- JP-A- H11 208 782
- JP-U- H0 643 552

## Description

### Technical Field

The present invention relates to an aseptic filling apparatus which sterilizes a preform, molds the sterilized preform into a bottle, fills sterilized content into the bottle in an aseptic atmosphere and, then, seals the bottle with a sterilized cap, wherein the aseptic filling apparatus includes an inspection unit which inspects the molded bottle in an aseptic atmosphere.

### Background Art

Conventionally, a sterilization method is proposed where sterilizer is applied by coating to a preform while the preform continuously travels, and the preform is introduced into a heating furnace in the coated state to heat the preform to a temperature for molding the preform into a container in the heating furnace so that applied sterilizer is dried and activated simultaneously by such heating (Patent Literatures 1 and 2). However, these Patent Literatures do not describe the inspection of a bottle obtained by molding a preform into the bottle.

On the other hand, a technique has been proposed where various inspections are performed before a bottle, obtained by molding a preform, is sterilized (Patent Literature 3). Abnormal bottles are removed due to the inspections, and content is filled only into normally molded bottles so that appropriate products can be supplied to the market. In this technique, bacteria in a molding unit which includes a molding device for molding a preform into a bottle, or sterilizer sprayed on a bottle in a sterilizing unit, which includes a sterilization device, may enter an inspection unit which includes inspection equipment. To prevent entrance of bacteria or sterilizer, a technique has been proposed where aseptic air is supplied to the inspection unit so as to keep the inspection unit at a positive pressure higher than that in the molding unit and the sterilizing unit. Particularly to prevent corrosion of the inspection equipment caused by flowing in of sterilizer, an atmosphere shut-off chamber is provided between the inspection unit and the sterilizing unit (Patent Literature 4).

A drink filling method has been also proposed where a preform is preheated, hydrogen peroxide mist or gas is sprayed on the preheated preform, the preform is further heated to a molding temperature, the preform attaining the molding temperature is molded into a bottle in a blow molding die which continuously travels in the same manner as the preform, and the bottle is removed from the blow molding die and, then, drink is filled into the bottle, and the bottle is sealed with a cap (see Patent Literatures 5 and 6). In the technique disclosed in Patent Literature 5, a preform is sterilized and is molded into a bottle and, thereafter, the bottle is inspected, and is also sterilized. In the technique disclosed in Patent Literature 6, only a preform is sterilized, and aseptic air is blown into a chamber, where a molding unit and an inspection unit are formed into an integral body, so as to maintain asepticity of the sterilized preform and a bottle obtained by molding the preform.

Usually, to ensure asepticity of the aseptic filling apparatus, a chamber of the aseptic filling apparatus which should be formed into an aseptic zone is sterilized before the aseptic filling apparatus is operated (Patent Literature 7). This is because the aseptic filling apparatus cannot exhibit the function thereof without sterilizing bacteria or the like which enter the aseptic zone of the aseptic filling apparatus before the aseptic filling apparatus is operated.

EP 3 070 010 A1 describes a method of sterilizing a preform includes steps of sterilizing bacteria adhering to a preform made of resin by gasifying a sterilizer, discharging a sterilized gas from a nozzle toward a preform, now traveling, so as to adhere to the preform; activating the sterilizer adhering to the preform by blasting hot air to the preform; and sterilizing bacteria adhering to the preform, and removing the sterilizer adhering to the preform, wherein the above steps are performed sequentially in order.

DE 10 2014 216576 A1 describes a container handling machine with a conveyor for transporting containers along a transport path in at least one container receptacle rotatable relative to the transport path, characterized in that the container handling machine has an inspection device with at least one camera for inspecting the container, and that the camera can be moved at least partially along the transport path with a separate moving unit, wherein the camera is placed in a protective housing.

EP 2 653 395 A1 describes a soft drink filling method including the steps of molding a bottle from a heated preform by a blow molding process, supplying hot water into the bottle at a time when the heat applied to the preform remains to thereby sterilize an interior of the bottle by making the hot water contact to an entire inner surface of the bottle, and filling the bottle with soft drink and then sealing the bottle.

DE 10 2004 054859 A1 describes an optical control of the presence of foam at the neck of a filled bottle, wherein the bottle neck is positioned between a light source and a camera.

### Citation List

### Patent Literature

Patent Literature 1: Japanese Patent Laid-Open No. 2008-183899
Patent Literature 2: Japanese Patent Laid-Open No. 2008-546605
Patent Literature 3: Japanese Patent Laid-Open No. 2010-155631
Patent Literature 4: Japanese Patent Laid-Open No. 2013-216391
Patent Literature 5: Japanese Patent Laid-Open No. 2013-35561
Patent Literature 6: Japanese Patent Laid-Open No. 2013-35562
Patent Literature 7: Japanese Patent Laid-Open No. 11-208782

### Summary of Invention

### Technical Problem

According to the above-mentioned prior arts, in the case where a preform is molded into a bottle and, thereafter, the bottle is sterilized, the molded bottle can be inspected in a non-aseptic zone. This is because even if bacteria or the like adhere to the bottle in the inspection unit, a sterilizing unit is disposed downstream of the inspection unit and hence, the bottle can be sterilized before content is filled into the bottle. However, inspection unit is disposed adjacently to the sterilizing unit and hence, it is necessary to prevent sterilizing gas or mist generated in the sterilizing unit from flowing into the inspection unit. The reason is to prevent inspection equipment in the inspection unit from being corroded by the sterilizer.

Conventionally, the aseptic filling apparatus for bottles sterilizes molded bottles. However, such a technique requires a large amount of sterilizer and increases the size of the apparatus. Accordingly, there is an increasing number of aseptic filling apparatuses which performs sterilization in the preform stage. However, after a bottle is sterilized in the preform stage, there is the task of maintaining asepticity of the bottle up to a filling unit which fills content. It is particularly necessary to prevent bacteria or the like from adhering to a bottle in the inspection unit after the bottle is molded. Patent Literature 6 proposes a technique where aseptic air is blown into an inspection unit during operation of the aseptic filling apparatus. Performing such an operation can prevent bacteria or the like from flowing into the inspection unit during operation. However, the inspection unit cannot be sterilized before the aseptic filling apparatus is operated and hence, there is a possibility that bacteria or the like which enter the inspection unit before the operation adheres to a bottle, or bacteria or the like adhering to the bottle are brought into the filling unit disposed downstream of the inspection unit, thus spreading contamination caused by the bacteria or the like. Accordingly, the inspection unit, which inspects a bottle molded after being sterilized in the preform stage, is required to be sterilized before the aseptic filling apparatus is operated.

The present invention has been made to overcome the above-mentioned problems, and it is an object of the present invention to provide an aseptic filling apparatus which performs sterilization on preforms, and which includes an inspection unit capable of maintaining asepticity with certainty.

### Solution to Problem

The underlying problem is solved by the aseptic filling apparatus having the features of claim 1. Preferred embodiments are defined in the dependent claims. The aseptic filling apparatus according to the present invention is characterized by including at least: An aseptic filling apparatus comprising at least: a sterilizing unit configured to sterilize a preform; a molding unit configured to mold a bottle by heating the preform; an inspection unit configured to inspect the bottle, the inspection unit including inspection equipment accommodated in an airtight container; a filling unit configured to fill content into the bottle; and a sealing unit configured to seal the bottle into which the content is filled.

In the aseptic filling apparatus according to the present invention, an inspection unit chamber configured to shield the inspection unit. It is preferable that, a sterilization device configured in the inspection unit chamber to sterilize an inside of the inspection unit chamber.

In the aseptic filling apparatus according to the present invention, an aseptic air supply device configured to keep an inside of the airtight container at a positive pressure.

In the aseptic filling apparatus according to the present invention, a heating device configured in the aseptic air supply device to heat an aseptic air, the aseptic air keeping the inside of the airtight container at a positive pressure.

It is preferable that, in the aseptic filling apparatus according to the present invention, a light transmitting window configured in at least a portion of a wall of the airtight container.

It is preferable that, in the aseptic filling apparatus according to the present invention, the inspection equipment include at least one or more of: first inspection equipment configured to inspect a barrel portion of the bottle; second inspection equipment configured to inspect a support ring of the bottle; third inspection equipment configured to inspect a top surface of a mouth portion of the bottle; and fourth inspection equipment configured to inspect a bottom portion of the bottle.

### Advantageous Effects of Invention

According to the present invention, in the aseptic filling apparatus which sterilizes a preform, the inspection unit, which inspects a bottle obtained by molding a preform, can be sterilized before the aseptic filling apparatus is operated and hence, asepticity of the aseptic filling apparatus can be ensured and maintained.

### Brief Description of Drawings

[FIG. 1] FIG. 1 is a plan view showing one example of the schematic configuration of an aseptic filling apparatus according to an embodiment of the present invention.
[FIG. 2] FIG. 2(A) and FIG. 2(B) are views showing a step of sterilizing a preform in the aseptic filling apparatus according to the embodiment of the present invention, wherein FIG. 2(A) shows a step of spraying a sterilizing gas on a preform, and FIG. 2(B) shows a step of blowing air into the preform.
[FIG. 3] FIG. 3 is a view showing a sterilizing gas generator incorporated in the aseptic filling apparatus according to the embodiment of the present invention.
[FIG. 4] FIG. 4 is a view showing an air blowing nozzle incorporated in the aseptic filling apparatus according to the embodiment of the present invention.
[FIG. 5] FIG. 5(C) to FIG. 5(G) are views showing steps in a molding unit of the aseptic filling apparatus according to the embodiment of the present invention, wherein FIG. 5(C) shows a step of heating a preform, FIG. 5(D) shows a step of conveying the preform to the molding unit, FIG. 5(E) shows a step of molding the preform into a bottle, FIG. 5(F) shows a step of taking out the molded bottle, and FIG. 5(G) shows a step of conveying the bottle to an inspection unit.
[FIG. 6] FIG. 6(H) to FIG. 6(K) are views showing inspections performed in the inspection unit of the aseptic filling apparatus according to the embodiment of the present invention, wherein FIG. 6(H) shows inspection of a barrel portion of the bottle, FIG. 6(I) shows inspection of a support ring, FIG. 6(J) shows inspection of a top surface of a mouth portion of the bottle, and FIG. 6(K) shows inspection of a bottom portion of the bottle.
[FIG. 7] FIG. 7 is a view showing one example of an airtight container which is incorporated in the inspection unit of the aseptic filling apparatus according to the embodiment of the present invention, and which accommodates inspection equipment.
[FIG. 8] FIG. 8 is a view showing one example of a sterilization device which is incorporated in an inspection unit chamber of the aseptic filling apparatus according to the embodiment of the present invention.
[FIG. 9] FIG. 9(L) and FIG. 9(M) are views showing steps in a filling unit and a sealing unit of the aseptic filling apparatus according to the embodiment of the present invention, wherein FIG. 9(L) shows a step of fill content into the bottle, and FIG. 9(M) shows a step of sealing the bottle into which the content is filled.

### Description of Embodiments

Hereinafter, an embodiment of the present invention will be described with reference to the drawings.

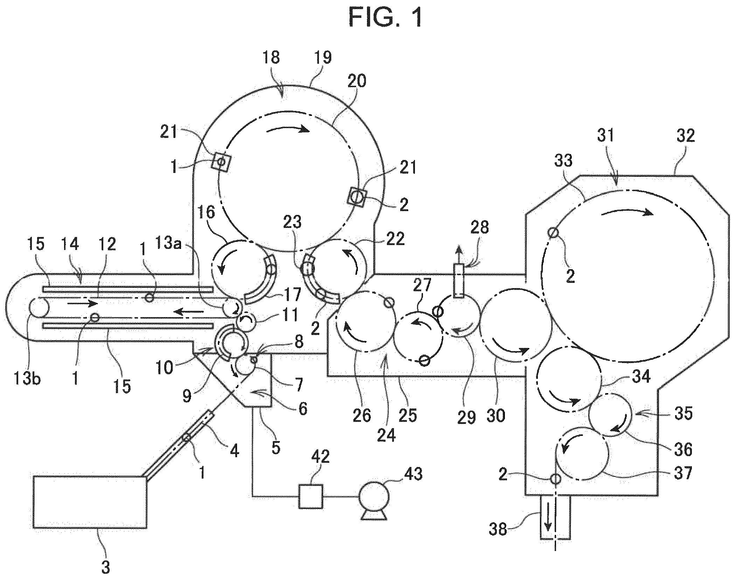

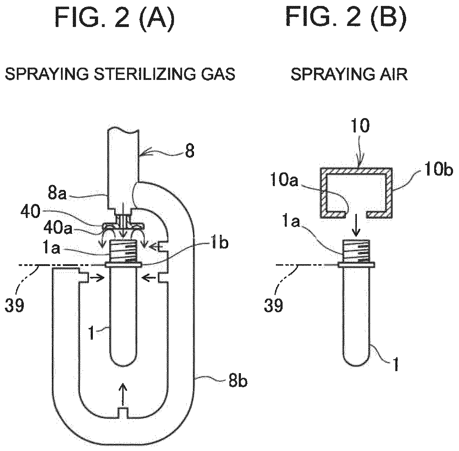

First, a summary of an aseptic filling apparatus is described with reference to FIG. 1, wherein the aseptic filling apparatus is formed of: a sterilizing unit which sterilizes a preform supplied from a preform supply device; a molding unit which molds the sterilized preform into a bottle; an inspection unit which performs an inspection on the molded bottle; a filling unit which fills content into the inspected bottle; and a sealing unit which seals the bottle into which the content is filled. The details of the respective components are described with reference to FIG. 2, FIG. 4, FIG. 5, FIG. 6, FIG. 8 and FIG. 9. According to this embodiment, the inspection unit can be sterilized before the aseptic filling apparatus, which sterilizes a preform, is operated and hence, asepticity of the aseptic filling apparatus can be maintained with certainty during operation.

### (Summary of aseptic filling apparatus)

As shown in FIG. 1, the aseptic filling apparatus according to this embodiment includes: a preform supply device 3 which supplies preforms 1; a sterilizing unit 6 which sterilizes the supplied preform 1; a molding unit 18 which molds a preform 1 into a bottle 2; an inspection unit 24 which inspects the molded bottle 2; a filling unit 31 which fills sterilized content into the inspected bottle 2; and a sealing unit 35 which seals the bottle 2, into which the content is filled, with a sterilized cap 61.

The sterilizing unit 6 which sterilizes the preform 1 is shielded by a sterilizing unit chamber 5. An air blowing portion, which blows air on the preform 1, and the molding unit 18, which molds the preform 1 into a bottle 2, are shielded by a molding unit chamber 19. The inspection unit 24, which inspects the bottle 2, is shielded by an inspection unit chamber 25. The filling unit 31, which fills content into the bottle 2, and the sealing unit 35, which seals the bottle 2 into which the content is filled, are shielded by a filling unit chamber 32.

Aseptic air, which is brought into an aseptic state by a aseptic filter, is supplied to the molding unit chamber 19, the inspection unit chamber 25, and the filling unit chamber 32 so that the inside of the respective chambers is kept at a positive pressure by the aseptic air during operation of the aseptic filling apparatus. Pressures are kept at a positive pressure, and are set such that the pressure in the filling unit chamber 32 assumes the highest pressure, and the pressure reduces toward the upstream direction from the inspection unit chamber 25 to the molding unit chamber 19 in that order. An exhaust device is coupled to the sterilizing unit chamber 5 for the preforms 1. The exhaust device is formed of a filter 42, which decomposes sterilizer in the air in the sterilizing unit chamber 5, and a blower 43. The air in the sterilizing unit chamber 5 is exhausted during operation of the aseptic filling apparatus and hence, it is possible to prevent sterilizer from flowing into the molding unit 18 which is disposed adjacently to the sterilizing unit chamber 5.

### (Detail of aseptic filling apparatus)

First, the preforms 1 shown in FIG. 2(A) are supplied from the preform supply device 3 shown in FIG. 1, and are continuously conveyed to the sterilizing unit 6, which sterilizes the preforms 1, by a preform conveyor 4 at a desired speed.

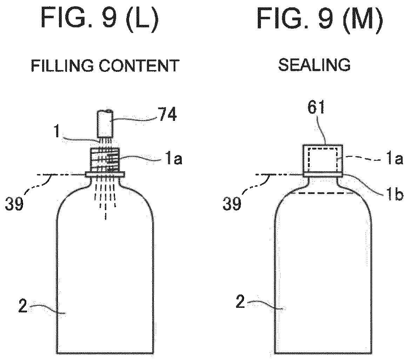

Each preform 1 in this embodiment is a bottomed tubular body similar to a test tube, and a mouth portion 1a similar to that of the bottle 2 shown in FIG. 9(M) is formed on the preform 1 in the early stage of the molding of the preform 1. A male thread is formed on the mouth portion 1a of the preform 1 simultaneously with the molding of the preform 1. A support ring 1b for conveyance is also formed on the preform 1 at the lower portion of the mouth portion 1a. The preform 1 or the bottle 2 travels through the aseptic filling apparatus while being held by a gripper 39 via the support ring 1b. The preform 1 is molded by injection molding, compression molding or the like. A material for forming the preform 1 may be a thermoplastic resin, such as polyethylene terephthalate, polyethylene naphthalate, polypropylene, or polyethylene. The material for forming the preform 1 may be a single substance or a mixture of these thermoplastic resins, or may contain a recycled thermoplastic resin. Further, in order to impart a barrier property, the material for forming the preform 1 may contain a thermoplastic resin, such as ethylene-vinyl alcohol copolymer, or polyamide, where aromatic amine, such as m-xylylenediamine, acts as a monomer, in the form of a layer or a mixture.

The preforms 1 are held by the grippers 39, provided on a sterilizing gas spraying wheel 7 at fixed intervals, thus being delivered to the sterilizing gas spraying wheel 7 from the preform conveyor 4. As shown in FIG. 2(A), sterilizing gas is sprayed on the delivered preform 1 by a sterilizing gas spraying nozzle 8 so as to sterilize the preform 1.

As shown in FIG. 2(A), the sterilizing gas flows while being divided into two streams in the sterilizing gas spraying nozzle 8. The sterilizing gas is sprayed toward the inside of the preform 1 from one nozzle 8a, and the sterilizing gas is sprayed toward the outer surface of the preform 1 from another nozzle 8b. After the sterilizing gas is discharged from the sterilizing gas spraying nozzle 8, the sterilizing gas flows into the preform 1 or comes into contact with the outer surface of the preform 1 in the form of a gas, a mist, or a mixture of gas and mist.

After sterilizing gas sprayed toward the inside of the preform 1 flows into the preform 1, the sterilizing gas flows out from the mouth portion 1a of the preform 1. The flow of the sterilizing gas or the like which flows out from the mouth portion 1a impinges on an umbrella-shaped member 40 and, then, the flow changes the direction toward the outer surface of the preform 1 while being guided by the inner surface of the umbrella-shaped member 40 whereby the flow comes into contact with the outer surface of the preform 1. Forming an annular groove 40a on the umbrella-shaped member 40 allows a sterilizing gas or the like which flows out from the mouth portion 1a to flow along the outer surface of the preform 1.

As described above, sterilizing gas or mist, or mixture of the sterilizing gas and sterilizing mist comes into contact with and adheres to the inner and outer surfaces of the preform 1. Accordingly, bacteria or the like adhering to the front surface of the preform 1 are sterilized.

The number of sterilizing gas spraying nozzles 8 shown in FIG. 2(A) is not limited to one. It may be configured such that a plurality of sterilizing gas spraying nozzles 8 is arranged along a traveling path for the preform 1, and a sterilizing gas is sprayed toward the preform 1 from these sterilizing gas spraying nozzles 8. Further, by varying the diameter of the sterilizing gas spraying nozzle 8, the nozzle 8a, or the nozzle 8b, or the diameter and the number of sterilizing gas blow-out ports formed on the nozzle 8b, it is possible to respectively adjust amounts of sterilizer adhering to the inner surface and the outer surface of the preform 1.

It may be also configured such that aseptic air at room temperature or heated aseptic air is supplied to the sterilizing gas spraying nozzle 8, the nozzle 8a, and the nozzle 8b from intermediate portions of these nozzles, and sterilizing gas which is diluted by aseptic air is sprayed on the preform 1.

Immediately before sterilizing gas is sprayed on the preform 1 shown in FIG. 2(A), the preform 1 may be preheated by blowing hot air on the preform 1. Performing this preheating allows sterilization effect of the preform 1 to be further improved.

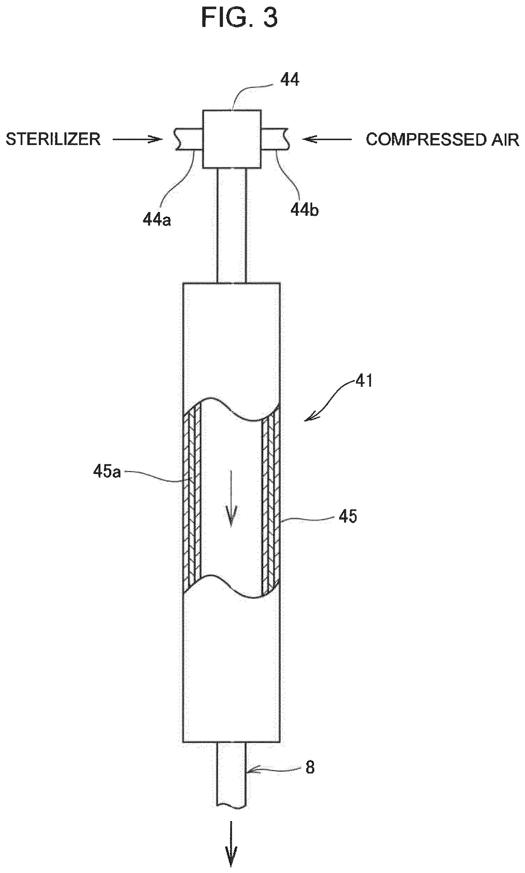

Sterilizing gas sprayed on the preform 1 is generated by a sterilizing gas generator 41 shown in FIG. 3. The sterilizing gas generator 41 includes a sterilizer supplying portion 44 and a vaporizing portion 45. The sterilizer supplying portion 44 is a twin-fluid spray nozzle which supplies sterilizer in the form of a droplet. The vaporizing portion 45 heats the sterilizer supplied from the sterilizer supplying portion 44 to a temperature lower than a decomposition temperature to vaporize the sterilizer. The sterilizer supplying portion 44 takes in sterilizer and compressed air respectively from a sterilizer supply path 44a and a compressed air supply path 44b to spray the sterilizer into the vaporizing portion 45. The vaporizing portion 45 is a pipe where a heater 45a is sandwiched between the inner and outer walls of the pipe. The vaporizing portion 45 heats sterilizer sprayed into the pipe to vaporize the sterilizer. The vaporized sterilizing gas is ejected to the outside of the vaporizing portion 45 from the sterilizing gas spraying nozzle 8. The vaporizing portion 45 may be heated by dielectric heating in place of utilizing the heater 45a.

With regard to operation conditions of the sterilizer supplying portion 44, for example, the pressure of compressed air is adjusted within a range from 0.05 MPa to 0.6 MPa. Further, sterilizer may fall under gravity or by applying a pressure. The supply amount of sterilizer may be set as desired, and the sterilizer may be supplied within a range from 1g/min to 100g/min, for example. Further, heating the inner surface of the vaporizing portion 45 to between 140°C and 450°C allows the sprayed sterilizer to be vaporized.

The ejected sterilizing gas or mist, or mixture of sterilizing gas and sterilizing mist is sprayed on the preform 1 from the sterilizing gas spraying nozzle 8 as shown in FIG. 2(A). The spray amount of sterilizing gas or mist, or mixture of sterilizing gas and sterilizing mist is arbitrarily determined. The spray amount is determined according to the amount of sterilizer supplied to the sterilizing gas generator 41 and a spray time. A plurality of sterilizing gas generators 41 may be provided. The spray amount varies also depending on the size of the preform 1. In the case where hydrogen peroxide solution is used as sterilizer, it is proper to set the amount of hydrogen peroxide to a value which falls within a range from 1×10⁻³g/mm² to 1g/mm². When the amount is less than 1×10⁻³g/mm², sterilization is not sufficiently performed. On the other hand, when the amount exceeds 1g/mm², the amount of hydrogen peroxide remaining on the preform 1 increases.

It is preferable that sterilizer contain at least hydrogen peroxide. It is proper to set the content of hydrogen peroxide to a value which falls within a range from 0.5% by mass to 65% by mass. When the content is less than 0.5% by mass, sterilizing power may not be sufficient. On the other hand, when the content exceeds 65% by mass, it becomes difficult to handle sterilizer in terms of safety. It is more preferable to set the content of hydrogen peroxide to a value which falls within a range from 0.5% by mass to 40% by mass. When the content of hydrogen peroxide is equal to or less than 40% by mass, sterilizer can be handled more easily, and have a low concentration and hence, it is possible to reduce the remaining amount of hydrogen peroxide after sterilization is performed.

Sterilizer contains water. The sterilizer may also contain one, two or more kinds selected from alcohols, such as methyl alcohol, ethyl alcohol, isopropyl alcohol, n-propyl alcohol, and butyl alcohol, ketones, such as acetone, methyl ethyl ketone, and acetylacetone, glycol ether and the like.

The sterilizer may further contain a compound having a sterilization effect, such as peracetic acid, acetic acid, chlorine compound, or ozone, or an additive, such as a cationic surfactant, a nonionic surfactant, or a phosphoric acid compound.

The preform 1 to which sterilizing gas is sprayed is delivered to an air blowing wheel 9 as shown in FIG. 1. As shown in FIG. 2(B), air which is brought into an aseptic state is blown by an air blowing nozzle 10 to the preform 1, which is delivered to the air blowing wheel 9, while the preform 1 is conveyed by the gripper 39.

Blowing air activates sterilizer adhering to the front surface of the preform 1 so that bacteria or the like which are present on the front surface of the preform 1 are sterilized. Further, blowing air rapidly removes sterilizer adhering to the preform 1 from the front surface of the preform 1. The sterilizer adhering to the preform 1 is removed from the preform 1 by blowing air before the preform 1 enters a heating furnace 14 and hence, it is possible to prevent sterilizer from damaging various kinds of equipment, such as a sealing member, in the molding unit 18. It is also possible to prevent the occurrence of defective molding, such as whitening, strain, or molding unevenness of a bottle, which is caused by sterilizer adhering to the preform 1.

Air blown on the preform 1 may have room temperature, or may be hot air obtained by heating air. However, it is preferable to use hot air. Using hot air promotes decomposition of sterilizer, thus improving a sterilization effect, and reducing a remaining amount of sterilizer. It is desirable that the temperature of hot air blown on the preform 1 be set to between 40°C and 140°C. When the temperature of hot air is lower than 40°C, only a small effect can be obtained by heating air. On the other hand, when the temperature of the preform 1 exceeds 70°C, a problem occurs, such as deformation of the mouth portion 1a of the preform 1. Accordingly, it is desirable that the temperature of hot air be lower than 140°C.

As shown in FIG. 2(B), air which is brought into an aseptic state is blown through a slit-shaped blow-out port 10a formed in a box-shaped manifold 10b forming a main body of the air blowing nozzle 10.

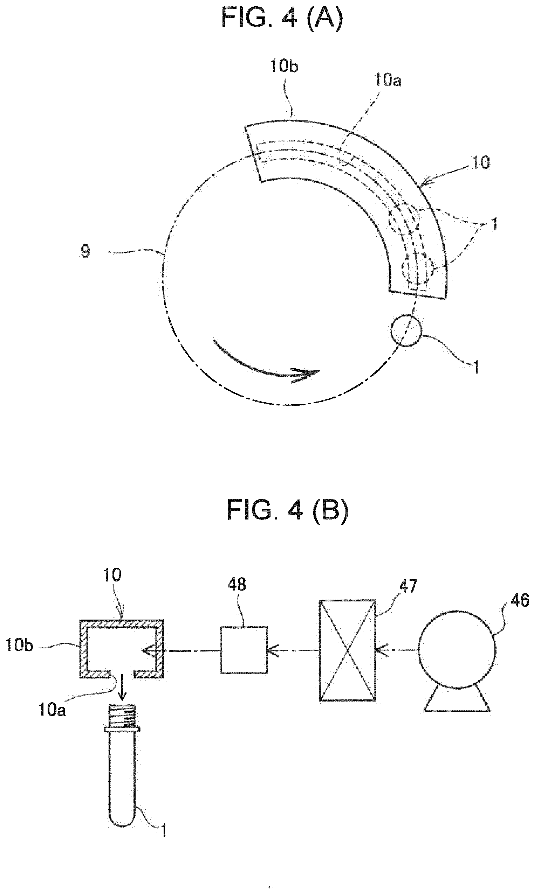

Further, as shown in FIG. 4(A), the air blowing nozzle 10 includes the box-shaped manifold 10b which curves along the arc of the air blowing wheel 9, and the air blowing nozzle 10 has the slit-shaped blow-out port 10a on the bottom surface of the manifold 10b. The air blowing nozzle 10 is disposed on the air blowing wheel 9 such that the blow-out port 10a extends along the traveling path for the preforms 1 in the air blowing wheel 9. As shown in FIG. 4(B), a blower 46, a aseptic filter 47, and an electric heater 48 are coupled to the manifold 10b. Outside air is taken in through the blower 46, and bacteria are removed from the outside air by the aseptic filter 47. Then, the outside air is heated by the electric heater 48 to become hot air, and the hot air is sent into the air blowing nozzle 10.

Air supplied to the air blowing nozzle 10 may not be air from the blower 46, but may be air obtained by bringing compressed air with a higher propulsive force into an aseptic state by a aseptic filter. Alternatively, high pressure air used in a molding machine 20 for blow molding may be recovered for reuse.

As shown in FIG. 2(B), air supplied into the manifold 10b of the air blowing nozzle 10 is ejected from the blow-out port 10a, thus being blown on the preform 1 traveling below the blow-out port 10a with the mouth portion 1a directing upward. A part of the blown air flows into the preform 1, and remaining portions of the blown air flows along the outer surface of the preform 1.

Air may be blown on the preform 1 such that air is blown toward the preform 1 from a cylindrical blowing nozzle. It may be also configured such that a suction pipe is disposed in the vicinity of the cylindrical blowing nozzle, and foreign substances, such as dust, which are discharged to the outside of the preform 1 are sucked by the suction pipe at the time of blowing air into the preform 1 from the cylindrical blowing nozzle. Collecting foreign substances using the suction pipe as described above can prevent foreign substances from flowing into another preform 1 or into a bottle 2 which is obtained by molding the preform 1. In the case where air is blown into the preform 1 from the cylindrical blowing nozzle, the configuration may be adopted where the preform 1 is held in an inverted position, and the cylindrical blowing nozzle is disposed while directing upward. In this case, foreign substances fall to the outside of the preform 1 due to wind pressure of blown air, and the dead weight of the foreign substances.

Air may be blown by an air blowing nozzle similar to the sterilizing gas spraying nozzle 8 shown in FIG. 2(A).

Blowing of air on the preform 1 is not absolutely necessary, and may not be performed. In such a case, after a sterilizing gas is sprayed on the preform 1, the preform 1 is heated without being subjected to any treatment. The inner surface and outer surface of the preform 1 to which the sterilizing gas is sprayed have sterilizer adhering to these surfaces. When the preform 1 is heated to a molding temperature, sterilizer adhering to the surfaces is activated so that the surfaces of the preform 1 are sterilized. Further, excess sterilizer is evaporated by being heated.

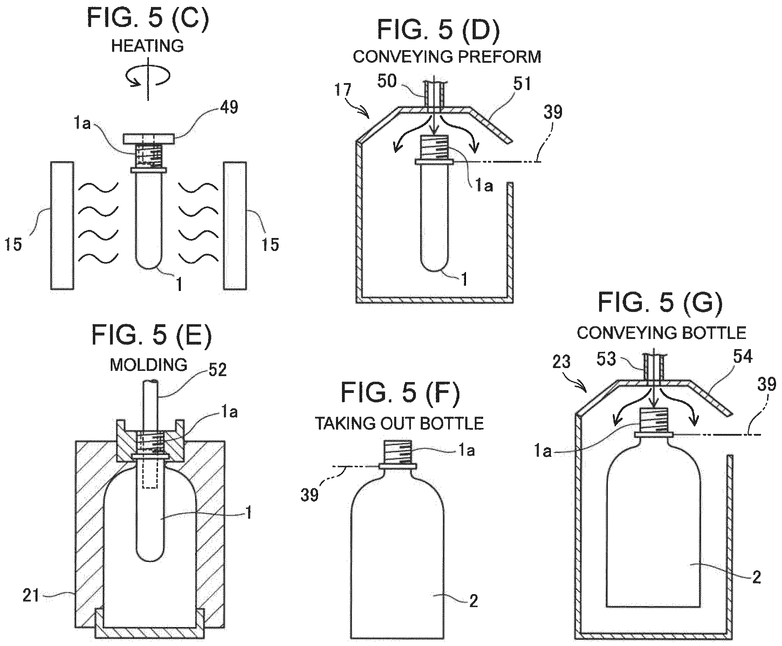

The preform 1 which is subjected to a sterilizing step is delivered to a wheel 11 shown in FIG. 1. At this point of operation, the preform 1 is released from the gripper 39 and, as shown in FIG. 5(C), a spindle 49 is inserted into the mouth portion 1a of the preform 1 and, then, the preform 1 is conveyed into the heating furnace 14 by an endless chain 12.

The preform 1 which enters the heating furnace 14 is, as shown in FIG. 5(C), heated to a temperature suitable for blow molding, which is performed thereafter, by an infrared heater 15 or another heating device. It is preferable to set this temperature to between 90°C and 130°C.

The temperature of the mouth portion 1a of the preform 1 is suppressed to a temperature of 70°C or below so as to prevent deformation and the like.

As shown in FIG. 5(C), the spindle 49 is inserted into the mouth portion 1a of the preform 1, and the preform 1 is conveyed through the heating furnace 14 while being rotated. The spindles 49 are provided on the endless chain 12 at fixed intervals. The endless chain 12 is rotated by pulleys 13a and 13b. It may be also possible to adopt the configuration where a mandrel is inserted into the preform 1 in place of the spindle 49, and the preform 1 is conveyed while being rotated in an inverted state.

The heated preform 1 is, as shown in FIG. 1, released from the spindle 49, and is delivered to the gripper 39 of a wheel 16. Then, the preform 1 is conveyed to the molding machine 20 while aseptic air is blown on the mouth portion 1a side of the preform 1 from a preform aseptic air supply device 17. Aseptic air is blown on the heated preform 1 so that the preform 1 is supplied to a die 21 of the molding machine 20 while maintaining asepticity.

Aseptic air blown on the heated preform 1 may be hot air. Blowing hot air on the preform 1 prevents lowering of the temperature of the preform 1.

FIG. 5(D) shows the detail of the preform aseptic air supply device 17. A preform tunnel 51 is provided on the conveying path for the heated preforms 1 on the wheel 16 so as to enclose the conveying path for the preforms 1. The preform tunnel 51 covers the mouth portion 1a of each preform 1 from above, and the ceiling portion of the preform tunnel 51 is formed into a roof shape having inclined surfaces. A preform aseptic air supply nozzle 50 is also provided on the ceiling portion such that the pipes are arranged in a row or the preform aseptic air supply nozzle 50 has a slit shape. The preform aseptic air supply nozzle 50 blows out aseptic air toward the mouth portion 1a of the preform 1. With such a configuration, aseptic air is efficiently supplied to the preform 1 so that the preform 1 can travel in the molding unit chamber 19 while maintaining asepticity.

The preform 1 is conveyed through the wheel 16 while maintaining asepticity by blowing aseptic air on the preform 1 and, as shown in FIG. 5(E), the preform 1 is accommodated in the die 21 of the molding machine 20 so as to be blow molded into the bottle 2. A plurality of dies 21 and a plurality of blow nozzles 52 are provided around the molding machine 20, and turns around the molding machine 20 at a fixed speed with the rotation of the molding machine 20. When the heated preform 1 arrives at the die 21, the die 21 sandwiches the preform 1. Subsequently, the blow nozzle 52 is inserted into the preform 1, and a gas, such as air, is blown into the preform 1 from the blow nozzle 52. Then, the bottom portion of the preform 1 is made to extend by an extension rod not shown in the drawing so that the bottle 2 is molded in the die 21.

After the bottle 2 is molded, the die 21 is opened to take off the bottle 2 by the gripper 39 of a wheel 22 as shown in FIG. 5(F). As shown in FIG. 1, aseptic air is blown on the mouth portion 1a side of the bottle 2 by a bottle aseptic air supply device 23 until the bottle 2 is conveyed to the inspection unit 24. The bottle 2 is conveyed by the wheel 22 while aseptic air is blown on the bottle 2 and hence, the bottle 2 can be conveyed to the inspection unit 24 without being contaminated by bacteria or the like.

FIG. 5(G) shows the detail of the bottle aseptic air supply device 23. A bottle tunnel 54 is provided on the conveying path on the wheel 22, which conveys the bottles 2, so as to enclose the conveying path for the bottles 2. The bottle tunnel 54 covers the mouth portion 1a of each bottle 2 from above, and the ceiling portion of the bottle tunnel 54 is formed into a roof shape having inclined surfaces. A bottle aseptic air supply nozzle 53 is also provided on the ceiling portion such that the pipes are arranged in a row or the bottle aseptic air supply nozzle 53 has a slit shape. The bottle aseptic air supply nozzle 53 blows out aseptic air toward the mouth portion 1a of the bottle 2. With such a configuration, aseptic air is efficiently supplied to the bottle 2 so that the bottle 2 can travel in the molding unit chamber 19 while maintaining asepticity.

The molding unit chamber 19 is sterilized before being operated. One example of a sterilization method may be a method where the inside of the molding unit chamber 19 is gas sterilized by air containing hydrogen peroxide at a concentration of equal to or less than 10 mg/L. Alternatively, a portion with which the preform 1 or the bottle 2 comes into contact may be irradiated by an UV lamp (ultraviolet sterilization). Further, the molding machine 20 and the inside of the molding unit chamber 19 may be sterilized as follows. Sterilizer in the form of liquid is introduced into the inside of the preform 1 by a method, such as a dropping or the like. Then, air or the like is blown into the preform 1 from the blow nozzle 52 while maintaining a state where the sterilizer remains in the preform 1 so as to diffuse the sterilizer to the molding machine 20 and in the molding unit chamber 19.

The bottle 2 is delivered to an inspection wheel 26 of the inspection unit 24 via the wheel 22. Only the bottle 2 which is confirmed to have no defects by the inspection is further conveyed to the filling unit 31. The bottles 2 is required to be inspected by the inspection unit 24 so as to prevent the use of the bottle 2 having an abnormality, such as defective molding, leading to the production of an unacceptable product. When an abnormality is found by the inspection, the bottle is discharged to the outside of the aseptic filling apparatus by a defective bottle discharge device 28 shown in FIG. 1.

The inspection unit 24 is sterilized before being operated so that the inspection unit 24 forms an aseptic zone, to which aseptic air is supplied during operation. In performing sterilization before the inspection unit 24 is operated, inspection equipment is accommodated in an airtight container 57 so as to prevent the inspection equipment, which inspects the bottle 2, from coming into contact with sterilizer. The inspection equipment is accommodated in order to prevent the inspection equipment from coming into contact with sterilizer, thus being corroded. That is, the inspection unit 24 includes the inspection equipment accommodated in the airtight container 57.

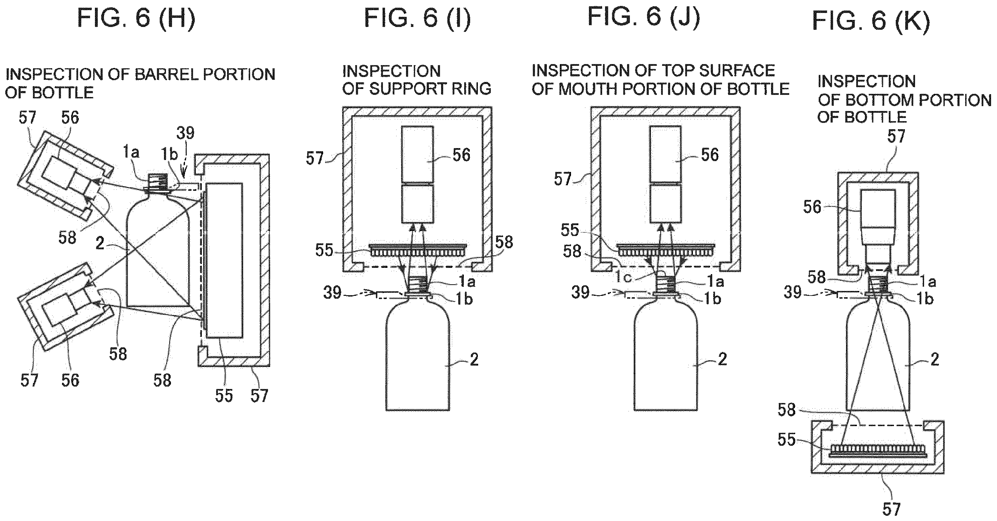

As shown in FIG. 6(H), first inspection equipment which inspects the barrel portion of a bottle is provided at a predetermined position around the inspection wheel 26 or a wheel 27. The first inspection equipment images the barrel portion of the bottle 2 having a circular cylindrical shape, an angular cylindrical shape or the like to determine whether the bottle 2 is acceptable or defective. To be more specific, the inspection equipment for a barrel portion of a bottle includes a lamp 55, which is an illumination device, and cameras 56, each of which is an imaging device. Illumination light emitted from the lamp 55 transmits the barrel portion of the bottle 2, and the cameras 56 image the barrel portion of the bottle 2 on receiving the light which transmits the bottle 2. The image of the barrel portion of the bottle 2 is processed by an image processing apparatus not shown in the drawing to determine whether or not abnormalities, such as flaws, foreign substances, or discoloration, are present. The bottle 2 having a flaw, foreign substances, discoloration or the like exceeding the allowable range is determined as a defective product.

When inspection equipment, such as the lamp 55 and the cameras 56, is installed on the inspection unit 24 without any covering, the inspection equipment is exposed to sterilizing gas or mist used for sterilization which is performed before the inspection unit 24 is operated. The sterilizer has a corroding effect on the materials of the inspection equipment and hence, the inspection equipment may deteriorate, thus causing problems in normal inspection. To prevent such a situation, the inspection equipment is accommodated in the airtight container 57 so as to prevent the inspection equipment from coming into contact with sterilizer.

The inspection equipment accommodated in the airtight container 57 and the image processing apparatus installed outside the aseptic filling apparatus exchange signals wirelessly or via a cable. The airtight container 57 is formed of curved surfaces, planar surfaces or the combination of the curved surfaces and the planar surfaces. Any material may be used as a material for forming the airtight container 57 provided that the material is no corroded or deteriorated by sterilizing mist or gas. For example, usually, it is preferable to use stainless steel or glass which is used for forming a member of the aseptic filling apparatus. Particularly, inspection is performed using light and hence, as shown in FIG. 6(H), a light transmitting window 58 made of a transparent material is provided at a portion where light passes through. It is preferable to use glass as the transparent material.

Inspection of the bottle 2 includes, in addition to the inspection of a barrel portion of a bottle shown in FIG. 6(H), inspection of a support ring shown in FIG. 6(I), inspection of a top surface of a mouth portion of the bottle shown in FIG. 6(J), and inspection of a bottom portion of the bottle shown in FIG. 6(K). The order of these inspections may be suitably changed. Another inspection may be added, or any of the inspections shown in FIG. 6 may be omitted. Inspection equipment used for each inspection is also accommodated in the airtight container 57 as shown in FIG. 6(I), FIG. 6(J), and FIG. 6 (K) in the same manner as the inspection equipment for a barrel portion of a bottle.

FIG. 6(I) shows the inspection of a support ring. The lamp 55 forms second inspection equipment which inspects the support ring 1b. The lamp 55 is provided above the support ring 1b while having an annular shape. For example, the lamp 55 is formed by arranging red LEDs in an annular shape. The lamp 55 may emit light in synchronism with the timing of imaging. A camera 56 is disposed to receive light which is illumination light of the lamp 55 reflected on the upper surface of the support ring 1b. The camera 56 includes a fisheye lens, for example, and converts an image captured by the fisheye lens into an electric signal by an imaging element, such as a CCD image sensor. Also in another inspection, the above-mentioned lamp 55 and the camera 56 are used as inspection equipment. A portion of the lamp 55 where reflected light passes through is a center portion of the lamp 55 having an annular shape. The lamp 55 is configured so as not to prevent reflected light from passing through the lamp 55. Further, equipment, such as the lamp 55 and the camera 56, is accommodated in the airtight container 57, and a portions of the airtight container 57 where illumination light and reflected light pass through is formed of the light transmitting window 58.

The camera 56 images the upper portion of the support ring 1b to inspect the state of the upper surface of the support ring 1b. The lower portion of the support ring 1b is held by the gripper 39 so that there is no possibility that the imaging of the support ring 1b is prevented by the gripper 39. The image of the support ring 1b is processed by the image processing apparatus not shown in the drawing to determine whether or not abnormalities, such as flaws or deformation, are present. In the case where the content is a drink, for example, there is a possibility that a purchaser of the bottle 2, which is a product, comes into contact with the support ring 1b at the time of opening the cap 61 shown in FIG. 9(M). Accordingly, when a flaw, deformation or the like exceeds the allowable range, the bottle 2 is determined as a defective product.

FIG. 6(J) shows the inspection of a top surface of a mouth portion of the bottle. The lamp 55 forms third inspection equipment which inspects a top surface 1c of the mouth portion 1a of the bottle 2. The lamp 55 is provided above the top surface 1c of the mouth portion 1a while having an annular shape. The camera 56 is disposed to receive light which is illumination light of the lamp 55 reflected on the upper surface of the top surface 1c of the mouth portion 1a. A portion of the lamp 55 where reflected light passes through is a center portion of the lamp 55 having an annular shape so that the lamp 55 does not prevent reflected light from passing through the lamp 55. Further, equipment, such as the lamp 55 and the camera 56, is accommodated in the airtight container 57, and a portion of the airtight container 57 where illumination light and reflected light pass through is formed of the light transmitting window 58.

An image imaged by the camera 56 is processed by the image processing apparatus not shown in the drawing to determine whether or not abnormalities, such as flaws or deformation, are present. The top surface 1c of the mouth portion 1a of the bottle is a portion which seals the bottle 2 by being brought into contact with the inner top surface of the cap 61 and hence, the top surface 1c is required to be flat and have smoothness. Accordingly, the bottle 2 having a flaw or deformation on the top surface 1c is determined as a defective product.

FIG. 6(K) shows the inspection of a bottom portion of the bottle. The lamp 55 forms fourth inspection equipment which inspects the bottom portion of the bottle 2. The lamp 55 is provided below the bottom portion of the bottle 2 while having an annular shape. A camera 56 is disposed above the mouth portion 1a of the bottle to receive light which is illumination light of the lamp 55 transmitting the bottom portion of the bottle 2. Each piece of equipment, such as the lamp 55 or the camera 56, is accommodated in the airtight container 57, and a portion of the airtight container 57 where illumination light and reflected light pass through is formed of the light transmitting window 58. In this case, the lamp 55 may not have an annular shape, but may have a circular shape.

The image of the bottom portion of the bottle 2 which is imaged by the camera 56 is processed by the image processing apparatus not shown in the drawing to determine whether or not abnormalities, such as flaws, foreign substances, or discoloration, are present. The bottle 2 having a flaw, foreign substances, discoloration or the like exceeding the allowable range is determined as a defective product.

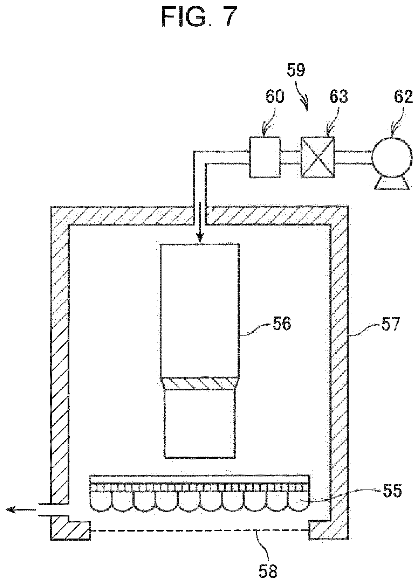

The airtight container 57 is configured such that sterilizer is prevented from flowing into the airtight container 57. To keep the inside of the airtight container 57 at a positive pressure, as shown in FIG. 7, an aseptic air supply device 59 is provided which brings air, taken in by a blower 62, into an aseptic state using an aseptic filter 63. Keeping the airtight container 57 at a positive pressure completely prevent inspection equipment from coming into contact with sterilizing mist or gas. The internal pressure of the airtight container 57 is set higher than the pressure of sterilizing gas or aseptic air which is introduced so as to sterilize the inspection unit chamber 25 before the aseptic filling apparatus is operated. For example, it is preferable that the internal pressure of the airtight container 57 be set to 1 to 100 Pa. Assume the case where the internal pressure of the airtight container 57 is less than 1 Pa. In such a case, when the airtight container 57 has a minute pin hole, flowing in of sterilizing mist or gas may not be prevented. Further, a pressure exceeding 100 Pa is an excessively large pressure for achieving the purpose of preventing flowing in of sterilizing mist or gas. Accordingly, such a pressure is not preferable. It may be configured such that aseptic air supplied to the airtight container 57 is discharged to the outside of the aseptic filling apparatus through an exhaust port formed in the airtight container 57 as shown in FIG. 7.

When aseptic air supplied at this time of operation is lower than the temperature of the atmosphere in the inspection unit chamber 25, condensation may occur on the outer surface of the airtight container 57. Even in the case where aseptic air is not supplied, when the temperature in the inside of the airtight container 57 is lower than that in the inspection unit chamber 25, condensation may occur on the outer surface of the airtight container 57. Accordingly, an aseptic air heating device 60 to the aseptic air supply device 59 is provided so as to heat aseptic air for preventing the temperature in the inside of the airtight container 57 from becoming lower than the temperature in the inspection unit chamber 25. This is because when condensation occurs on the light transmitting window 58, an erroneous inspection result may occur. It is preferable that the temperature of aseptic air supplied to the airtight container 57 be set to between 30°C and 50°C. When the temperature is lower than 30°C, condensation may occur on the outer surface of the airtight container 57. On the other hand, a temperature exceeding 50°C is excessively high for preventing the condensation. Aseptic air may be supplied to the airtight container 57 not only at the time of sterilizing the inspection unit chamber 25 before the aseptic filling apparatus is operated, but also during operation of the aseptic filling apparatus. With such a configuration, it is also possible to prevent the condensation on the outer surface of the airtight container 57 during operation.

When the bottle 2 inspected in the inspection wheels 26 and 27 is determined as a defective product, the bottle 2 is discharged to the outside of the inspection unit chamber 25 by the defective bottle discharge device 28 provided on a wheel 29. The bottle 2 which is determined as a defective product is not conveyed to the filling unit 31.

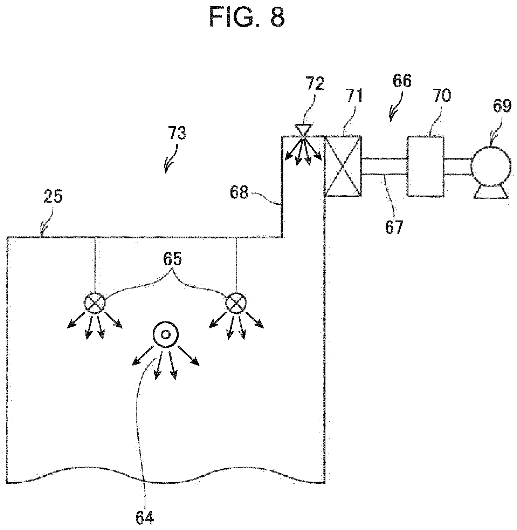

The inside of the inspection unit chamber 25, which shields the inspection unit 24, is sterilized before the aseptic filling apparatus is operated. Accordingly, an inspection unit chamber sterilization device 73 shown in FIG. 8 is provided in the aseptic filling apparatus. As shown in FIG. 8, the inspection unit chamber sterilization device 73 is formed of a sterilizer spray nozzle 64, heated water spray nozzles 65, and an inspection unit chamber aseptic air supply device 66.

For the sterilizer spray nozzle 64, a twin-fluid spray is used which sprays sterilizer in the form of a mixture with compressed air. The sterilizer spray nozzle 64 sprays sterilizer so as to cause the sterilizer to adhere to the entire area of the inspection unit chamber. The inspection unit chamber 25 is sterilized by the sprayed sterilizer. The sterilizer spray nozzle 64 is disposed such that sterilizer adheres to the entire area in the inspection unit chamber 25. It is preferable that sterilizer similar to sterilizer used for sterilizing the preform 1 can be used as sterilizer, and sterilizer containing peracetic acid or hydrogen peroxide be used. Sterilizer may be sprayed such that different sterilizers are sprayed a plurality of times.

After sterilizer is sprayed from the sterilizer spray nozzle 64, heated water is injected by the heated water spray nozzles 65 such that heated water is sprayed on the entire area of the inspection unit chamber 25. The heated water rinses sterilizer remaining in the inspection unit chamber 25. The heated water spray nozzles 65 are disposed so as to allow heated water to be sprayed on the entire area of the inspection unit chamber 25. For heated water, water which is heated at 121°C or above for 4 minutes or more, or water which is made to pass through the aseptic filter, thus being brought into an aseptic state is used. It is preferable that heated water sprayed on the inspection unit chamber 25 from the heated water spray nozzles 65 be heated to between 60°C and 100°C. For the heated water spray nozzle 65, a spray nozzle which uses a spinning ball is used, for example.

The inspection unit chamber aseptic air supply device 66 removes the heated water which is sprayed from the heated water spray nozzles 65, and which remains in the inspection unit chamber 25 vaporizing the heated water. Further, the inspection unit chamber aseptic air supply device 66 supplies aseptic air into the inspection unit chamber 25 so as to maintain asepticity in the inspection unit chamber 25 during operation of the aseptic filling apparatus. The duct of the inspection unit chamber aseptic air supply device 66 is connected to the ceiling of the inspection unit chamber 25. As shown in FIG. 8, the duct is provided with a horizontal portion 67 and a vertical portion 68 which extends downward toward the ceiling of the inspection unit chamber 25. The horizontal portion 67 is provided with a blower 69, a heating device 70, and a aseptic filter 71 in a direction from the upstream side toward the downstream side.

After air from the blower 69 is heated by the heating device 70, and bacteria are removed from the air by the aseptic filter 71, the air forms aseptic air, and is supplied into the inspection unit chamber 25. During operation of the aseptic filling apparatus, the inspection unit chamber 25 is held at a positive pressure by aseptic air, and aseptic air flowing out from the inspection unit chamber 25 flows into the molding unit chamber 19. The molding unit chamber 19 is also held at a positive pressure by aseptic air in the same manner as the filling unit chamber 32. However, the pressure in the filling unit chamber 32 is set at the highest pressure, and pressures in the chambers reduce in the order from the inspection unit chamber 25 to the molding unit chamber 19. For example, the inside of the filling unit chamber 32 is set to 30 to 100 Pa, the inside of the inspection unit chamber 25 is set to 10 to 30 Pa, and the inside of the molding unit chamber 19 is set to 0 to 10 Pa. Further, the sterilizing unit chamber 5 where sterilizing gas is sprayed on the preform 1 is exhausted and hence, the sterilizing unit chamber 5 is set to -30 to 0 Pa.

In performing sterilization before the aseptic filling apparatus is operated, sterilizer is sprayed by a sterilizer spray nozzle 72 so that the vertical portion 68 of the duct, the front surface of the aseptic filter 71, and the inner surface side of the inspection unit chamber 25 are sterilized. Heated water sprayed from the heated water spray nozzles 65 can also sterilize the vertical portion 68 of the duct, the front surface of the aseptic filter 71, and the inner surface of the inspection unit chamber 25 in the same manner.

The order and the number of times of spraying of sterilizer, spraying of heated water, and supplying of aseptic air may be determined as desired provided that the inspection unit chamber 25 is sterilized.

In the sterilization of the inspection unit chamber 25 performed before the aseptic filling apparatus is operated, the airtight container 57 protects inspection equipment from spraying of sterilizer, spraying of heated water, and supplying of heated aseptic air. As a result, it is possible to prevent inspection equipment from deteriorating or being damaged so that accuracy of the inspection result can be ensured.

The bottle 2 which is not determined as a defective product by the inspection is delivered to a filling wheel 33 of the filling unit 31 via a wheel 30. A large number of filling nozzles 74 are provided on the outer periphery of the filling wheel 33 for filling content sterilized by a sterilization device not shown in the drawing. As shown in FIG. 9(L), sterilized content is filled, by the filling nozzle 74, into the bottle 2 held by the gripper 39 in the filling wheel 33.

The bottle 2 into which the content is filled is delivered to a sealing wheel 36 of the sealing unit 35 via a wheel 34. A large number of sealing devices are provided on the outer periphery of the sealing wheel 36, and the sealing device performs capping with the cap 61 sterilized by a sterilization device not shown in the drawing. As shown in FIG. 9(M), in the sealing wheel 36, the sterilized cap 61 is mounted on the mouth portion 1a so that the bottle 2 held by the gripper 39 is sealed.

The sealed bottle 2 is placed on a conveyor not shown in the drawing via a wheel 37, and is discharged to the outside of the aseptic filling apparatus via a discharge chamber 38.

In the same manner as the inspection unit chamber 25, the filling unit chamber 32 is provided with a sterilization device as shown in FIG. 8. The filling unit chamber 32 is sterilized before the aseptic filling apparatus is operated in the same manner as the inspection unit chamber 25.

The present invention is configured as described above. However, the present invention is not limited to the above-mentioned embodiment, and various modifications are conceivable, wherein the invention is defined by the following claims.

### Reference Signs List

- 1:: preform
- 2:: bottle
- 5:: sterilizing unit chamber
- 8:: sterilizing gas spraying nozzle
- 10:: air blowing nozzle
- 17:: preform aseptic air supply device
- 18:: molding unit
- 19:: molding unit chamber
- 20:: molding machine
- 23:: bottle aseptic air supply device
- 24:: inspection unit
- 25:: inspection unit chamber
- 31:: filling unit
- 32:: filling unit chamber
- 35:: sealing unit
- 39:: gripper
- 41:: sterilizing gas generator
- 57:: airtight container
- 58:: light transmitting window
- 59:: aseptic air supply device
- 60:: aseptic air heating device
- 61:: cap
- 73:: inspection unit chamber sterilization device

## Claims

1. An aseptic filling apparatus comprising at least:
a sterilizing unit (6) configured to sterilize a preform (1);
a molding unit (18) configured to mold a bottle (2) by heating the preform (1);
an inspection unit (24) configured to inspect the bottle (2);
a filling unit (31) configured to fill content into the bottle (2); and
a sealing unit (35) configured to seal the bottle (2) into which the content is filled;
**characterized in that**
the inspection unit (24) includes inspection equipment accommodated in an airtight container (57), and
an aseptic air supply device (59) is provided for keeping the airtight container (57) at a positive pressure,
wherein the airtight container (57) is configured such that sterilizer is prevented from flowing into the airtight container (57) by keeping the inside of the airtight container (57) at a positive pressure,
wherein the aseptic air supply device (59) is configured to bring air, taken in by a blower (62), into an aseptic state using an aseptic filter (63) therewith completely preventing the inspection equipment from coming into contact with sterilizing mist or gas which is introduced so as to sterilize an inspection unit chamber (25) before the aseptic filling apparatus is operated,
wherein an aseptic heating device (60) is provided to the aseptic air supply device (59) so as to heat aseptic air for preventing the temperature in the inside of the airtight container (57) from becoming lower than the temperature in the inspection unit chamber (25) to prevent condensation on a light transmitting window (58).

2. The aseptic filling apparatus according to claim 1, further comprising a sterilization device (73) configured in the inspection unit chamber (25) to sterilize an inside of the inspection unit chamber (25).

3. The aseptic filling apparatus according to any one of claims 1 or 2, further comprising a light transmitting window (58) configured in at least a portion of a wall of the airtight container (57).

4. The aseptic filling apparatus according to any one of claims 1 to 3, wherein
the inspection equipment includes at least one or more of: first inspection equipment configured to inspect a barrel portion of the bottle (2); second inspection equipment configured to inspect a support ring (1b) of the bottle (2); third inspection equipment configured to inspect a top surface of a mouth portion (1a) of the bottle (2); and fourth inspection equipment configured to inspect a bottom portion of the bottle (2).

## Patentansprüche

1. Eine aseptische Abfüllvorrichtung, umfassend zumindest:
eine Sterilisationseinheit (6), die dazu konfiguriert ist, eine Vorform (1) zu sterilisieren;
eine Formungseinheit (18), die dazu konfiguriert ist, durch Erhitzen der Vorform (1) eine Flasche (2) zu formen;
eine Inspektionseinheit (24), die dazu konfiguriert ist, die Flasche (2) zu inspizieren;
eine Abfülleinheit (31), die dazu konfiguriert ist, Inhalt in die Flasche (2) einzufüllen; und
eine Versiegelungseinheit (35), die dazu konfiguriert ist, die Flasche (2), in welche der Inhalt eingefüllt ist, zu versiegeln;
**dadurch gekennzeichnet, dass**
die Inspektionseinheit (24) eine Inspektionsausrüstung beinhaltet, die in einem luftdichten Behälter (57) untergebracht ist, und
eine aseptische Luftversorgungseinrichtung (59) bereitgestellt wird, um den luftdichten Behälter (57) auf einem positiven Druck zu halten,
wobei der luftdichte Behälter (57) derart konfiguriert ist, dass durch das Halten des Inneren des luftdichten Behälters (57) auf einem positiven Druck verhindert wird, dass Sterilisationsmittel in den luftdichten Behälter (57) strömt,
wobei die aseptische Luftversorgungseinrichtung (59) dazu konfiguriert ist, Luft, die von einem Gebläse (62) angesaugt wird, unter Verwendung eines aseptischen Filters (63) in einen aseptischen Zustand zu versetzen, wodurch vollständig verhindert wird, dass die Inspektionsausrüstung mit Sterilisationsnebel oder -gas in Kontakt kommt, der bzw. das eingeführt wird, um eine Inspektionseinheitskammer (25) vor dem Betrieb der aseptischen Abfüllvorrichtung zu sterilisieren,
wobei eine aseptische Heizvorrichtung (60) an der aseptischen Luftversorgungseinrichtung (59) bereitgestellt wird, um aseptische Luft zu erwärmen, damit verhindert wird, dass die Temperatur im Inneren des luftdichten Behälters (57) niedriger wird als die Temperatur in der Inspektionseinheitskammer (25), um Kondensation an einem lichtdurchlässigen Fenster (58) zu verhindern.

2. Die aseptische Abfüllvorrichtung nach Anspruch 1, ferner umfassend eine Sterilisationsvorrichtung (73), die in der Inspektionseinheitskammer (25) angeordnet ist, um das Innere der Inspektionseinheitskammer (25) zu sterilisieren.

3. Die aseptische Abfüllvorrichtung nach irgendeinem der Ansprüche 1 oder 2, ferner umfassend ein lichtdurchlässiges Fenster (58), das zumindest in einem Abschnitt einer Wand des luftdichten Behälters (57) angeordnet ist.

4. Die aseptische Abfüllvorrichtung nach irgendeinem der Ansprüche von 1 bis 3, wobei
die Inspektionsausrüstung zumindest ein oder mehrere der folgenden Elemente beinhaltet: eine erste Inspektionsausrüstung, die konfiguriert ist, um einen Mantelabschnitt der Flasche (2) zu inspizieren; eine zweite Inspektionsausrüstung, die konfiguriert ist, um einen Stützring (1b) der Flasche (2) zu inspizieren; eine dritte Inspektionsausrüstung, die konfiguriert ist, um eine Oberseite eines Mündungsabschnitts (1a) der Flasche (2) zu inspizieren; und eine vierte Inspektionsausrüstung, die konfiguriert ist, um einen Bodenabschnitt der Flasche (2) zu inspizieren.

## Revendications

1. Un dispositif de remplissage aseptique comprenant au moins :
une unité de stérilisation (6) configurée pour stériliser une préforme (1) ;
une unité de moulage (18) configurée pour mouler une bouteille (2) en chauffant la préforme (1) ;
une unité d'inspection (24) configurée pour inspecter la bouteille (2) ;
une unité de remplissage (31) configurée pour remplir un contenu dans la bouteille (2) ; et
une unité de scellage (35) configurée pour sceller la bouteille (2) dans laquelle le contenu est rempli ;
**caractérisé en ce que**
l'unité d'inspection (24) inclut un équipement d'inspection logé dans un conteneur étanche à l'air (57), et que
un dispositif d'alimentation en air aseptique (59) est prévu pour maintenir le conteneur étanche à l'air (57) à une pression positive,
sachant que le conteneur étanche à l'air (57) est configuré de manière que l'agent de stérilisation soit empêché de s'écouler dans le conteneur étanche à l'air (57) en maintenant l'intérieur du récipient étanche à l'air (57) à une pression positive,
sachant que le dispositif d'alimentation en air aseptique (59) est configuré pour amener de l'air, aspiré par un ventilateur (62), dans un état aseptique à l'aide d'un filtre aseptique (63), empêchant ainsi complètement l'équipement d'inspection d'entrer en contact avec le brouillard ou le gaz stérilisant qui est introduit afin de stériliser une chambre d'unité d'inspection (25) avant que le dispositif de remplissage aseptique ne soit mis en fonctionnement,
sachant qu'un dispositif de chauffage aseptique (60) est prévu sur le dispositif d'alimentation en air aseptique (59) afin de chauffer de l'air aseptique pour empêcher que la température à l'intérieur du récipient étanche à l'air (57) ne devienne inférieure à la température dans la chambre d'unité d'inspection (25) afin d'éviter une condensation sur une fenêtre transmissive à la lumière (58).

2. Le dispositif de remplissage aseptique d'après la revendication 1, comprenant en outre un dispositif de stérilisation (73) disposé dans la chambre d'unité d'inspection (25) pour stériliser l'intérieur de la chambre d'unité d'inspection (25).

3. Le dispositif de remplissage aseptique d'après l'une quelconque des revendications 1 ou 2, comprenant en outre une fenêtre transmissive à la lumière (58) disposée dans au moins une portion d'une paroi du récipient étanche à l'air (57).

4. Le dispositif de remplissage aseptique d'après l'une quelconque des revendications de 1 à 3, sachant que
l'équipement d'inspection inclut au moins un ou plusieurs des éléments suivants : un premier équipement d'inspection configuré pour inspecter une portion de corps de la bouteille (2) ; un deuxième équipement d'inspection configuré pour inspecter un anneau de support (1b) de la bouteille (2) ; un troisième équipement d'inspection configuré pour inspecter une surface supérieure d'une portion de goulot (1a) de la bouteille (2) ; et un quatrième équipement d'inspection configuré pour inspecter une partie inférieure de la bouteille (2).
